# EUROPEAN PATENT SPECIFICATION

(11) **EP 1 656 555 B1**
(45) Date of publication and mention of the grant of the patent: **21.03.2012**
(21) Application number: 04781634.3
(22) Date of filing: 19.08.2004
(51) Int. Cl.: G01N 33/53, A61K 38/00

(54) **METHODS AND COMPOSITIONS FOR MEASURING BIOLOGICALLY ACTIVE NATRIURETIC PEPTIDES AND FOR IMPROVING THEIR THERAPEUTIC POTENTIAL**
VERFAHREN UND ZUSAMMENSETZUNGEN ZUR MESSUNG BIOLOGISCH AKTIVER NATRIURETISCHER PEPTIDE UND ZUR VERBESSERUNG IHRES THERAPEUTISCHEN POTENTIALS
METHODES ET COMPOSITIONS PERMETTANT DE MESURER LES PEPTIDES NATRIURETIQUES ET D'AMELIORER LEUR POTENTIEL THERAPEUTIQUE

(30) Priority: 20.08.2003 US 645874; 04.02.2004 US 542086 P
(43) Date of publication of application: 17.05.2006
(73) Proprietor: Alere San Diego, Inc., San Diego, CA 92121 (US)
(72) Inventor: BUECHLER, Kenneth, F., Rancho Sant Fe, CA 92067 (US); WHITTAKER, Michael, San Diego, CA 92127 (US); REYNOLDS, Mark, Carlsbad, CA 92009 (US); SCOTT, Christopher, A., San Diego, CA 92122 (US)
(74) Representative: Schiweck, Weinzierl & Koch
(86) International application number: PCT/US2004/026984
(87) International publication number: WO 2005/019819

(56) References cited:
- WO-A-03/002553
- US-A- 5 786 163
- CORTI R ET AL: "Vasopeptidase inhibitors: a new therapeutic concept in cardiovascular disease?" CIRCULATION 9 OCT 2001, vol. 104, no. 15, 9 October 2001 (2001-10-09), pages 1856-1862, XP002432378 ISSN: 1524-4539
- JONES BARRY ET AL: "Hematopoietic stimulation by a dipeptidyl peptidase inhibitor reveals a novel regulatory mechanism and therapeutic treatment for blood cell deficiencies." BLOOD, vol. 102, no. 5, 8 May 2003 (2003-05-08), pages 1641-1648, XP002432379 ISSN: 0006-4971
- WALTHER T ET AL: "BIOCHEMICAL ANALYSIS OF NEUTRAL ENDOPEPTIDASE ACTIVITY REVEALS INDEPENDENT CATABOLISM OF ATRIAL AND BRAIN NATRIURETIC PEPTIDE" BIOLOGICAL CHEMISTRY, XX, XX, vol. 385, no. 2, February 2004 (2004-02), pages 179-184, XP009060566 ISSN: 1431-6730

## Description

### FIELD OF THE INVENTION

The present invention relates to medical diagnostics and therapeutics.

### BACKGROUND OF THE INVENTION

The following discussion of the background of the invention is merely provided to aid the reader in understanding the invention and is not admitted to describe or constitute prior art to the present Invention.

Natriuretic peptides are a group of naturally occurring substances that act in the body to oppose the activity of the renin-angiotensin system. There are three major natriuretic peptides: atrial natriuretic peptide (ANP), which is synthesized in the atria; brain-type natriuretic peptide (BNP), which is synthesized in the ventricles; and C-type natriuretic peptide (CNP), which is synthesized in the brain.

Mature human A-type natriuretic peptide (ANP) (also referred to as atrial natriuretic peptide) is a biologically active 28 amino acid peptide that is synthesized, stored, and released by atrial myocytes in response to atrial distension, angiotensin II stimulation, endothelin, and sympathetic stimulation (beta-adrenoceptor mediated). Mature ANP is generated by proteolytic cleavage of a 128 amino acid precursor molecule (pro-ANP), yielding the biologically active 28 amino acid peptide representing amino acids 99-126 of the pro-ANP molecule (ANP₉₉₋₁₂₆). Linear peptide fragments from the N-terminal prohormone segment have also been reported to have biological activity.

Mature human B-type natriuretic peptide (BNP) (also called brain-type natriuretic peptide) is a 32 amino acid, 4 kDa biologically active peptide that is involved in the natriuresis system to regulate blood pressure and fluid balance (Bonow, R.O., Circulation 93:1946-1950, 1996). The mature BNP hormone is generated by proteolytic cleavage of a 108-amino acid precursor molecule, referred to herein as "pro-BNP." Cleavage generates a 76-amino acid N-terminal peptide (amino acids 1-76), referred to as "NT pro BNP," and the 32-amino acid mature hormone, referred to as BNP or BNP 32 (amino acids 77-108). It has been suggested that each of these species -NT pro-BNP, BNP-32, and the pro-BNP - can circulate in human plasma (Tateyama et al., Biochem. Biophys. Res. Commun. 185:760-7, 1992; Hunt et al., Biochem. Biophys. Res. Commun. 214:1175-83, 1995).

Mature human C-type natriuretic peptide (CNP) a 22-amino acid peptide that is the primary active natriuretic peptide in the human brain; CNP is also considered to be an endothelium-derived relaxant factor, which acts in the same way as nitric oxide (NO) (Davidson et al., Circulation 93:1155-9, 1996). CNP is structurally related to A-type natriuretic peptide (ANP) and B-type natriuretic peptide (BNP); however, while ANP and BNP are synthesized predominantly in the myocardium, CNP is synthesized in the vascular endothelium as a precursor (pro-CNP) (Prickett et al., Biochem. Biophys. Res. Commun. 286:513-7, 2001). CNP is thought to possess vasodilator effects on both arteries and veins and has been reported to act mainly on the vein by increasing the intracellular cGMP concentration in vascular smooth muscle cells.

ANP and BNP are released in response to atrial and ventricular stretch, respectively, and will cause vasorelaxation, inhibition of aldosterone secretion in the adrenal cortex, and inhibition of renin secretion in the kidney. Both ANP and BNP will cause natriuresis and a reduction in intravascular volume, effects amplified by the antagonism of antidiuretic hormone (ADH). The physiologic effects of CNP differ from those of ANP and BNP; CNP has a hypotensive effect, but no significant diuretic or natriuretic actions. Increased blood levels of natriuretic peptides have been found in certain disease states, suggesting a role in the pathophysiology of those diseases, including stroke, congestive heart failure (CHF), cardiac ischemia, systemic hypertension, and acute myocardial infarction. *See*, *e.g.*, WO 02/089657; WO 02/083913; and WO 03/016910.
Numerous non-human homologs of the natriuretic peptides are known to those of skill in the art.

The natriuretic peptides, alone, collectively, and/or together with additional proteins, can serve as disease markers and indicators of prognosis in various cardiovascular conditions. For example, BNP, which is synthesized in the cardiac ventricles and correlates with left ventricular pressure, amount of dyspnea, and the state of neurohormonal modulation, makes this peptide the first potential marker for heart failure. Measurement of plasma BNP concentration is evolving as a very efficient and cost effective mass screening technique for identifying patients with various cardiac abnormalities regardless of etiology and degree of LV systolic dysfunction that can potentially develop into obvious heart failure and carry a high risk of a cardiovascular event. Finding a simple blood test that would aid in the diagnosis and management of patients with CHF clearly would have a favorable impact on the staggering costs associated with the disease.

Removal of the natriuretic peptides from the circulation is affected mainly by binding to clearance receptors and enzymatic degradation in the circulation. *See*, *e.g.*, Cho et al., Heart Dis. 1: 305-28, 1999;. Smith et al., J. Endocrinol. 167: 239-46, 2000. Additionally, human pro-BNP is reported to be processed in serum such that circulating pro-BNP is unlikely to be the intact 108 amino acid form. Hunt et al., Peptides 18:1475-81,1997. Degradation of the natriuretic peptides is believed to be mediated by neutral endopeptidase. For example, Norman et al. (Biochem. Biophys. Res. Commun. 28: 175: 22-30, 1991) report that neutral endopeptidase can cleave human BNP between residues 2 and 3, between residues 4 and 5, and between residues 17 and 18. Similarly, Lindberg and Andersson (Regul. Pept. 47: 53-63, 1993) report that human ANP is cleaved between residues 3 and 4 and residues 14 and 15. The biological activity of this hydrolyzed product was about 500-fold less than intact ANP. Additionally, Knecht et al. (Life Sci. 71: 2701-12, 2002) report that renal neutral endopeptidase is upregulated in heart failure, a condition where natriuretic peptide levels are increased. For this reason, neutral endopeptidase has been targeted for inhibition in treatment of cardiovascular disease. *See*, *e.g.*, Corti et al., Circulation 104: 1856-62, 2001.

Confusion over the stability of the natriuretic peptides, particularly in blood-derived samples (e.g., serum, plasma, whole blood) has been reported. ANP is reported to be a better substrate for neutral endopeptidase than is BNP. Similarly, Shimizu et al. (Clin. Chem, Acta 305: 181-6, 2001), Gobinet-Georges et al. (Clin. Chem. Lab. Med. 38: 519-23, 2000) and Murdoch et al. (Heart 78: 594-7, 1997) report that BNP is stable in certain blood-derived samples or when blood is collected under certain conditions. A more recent report by Shimizu et al. (Clin. Chem. Acta 316: 129-35, 2002) indicates that 94% of BNP in whole blood was a digested form in which 2 amino terminal residues had been removed; and that BNP in plasma was degraded to a number of unidentified forms.

### BRIEF SUMMARY OF THE INVENTION

It is one object of the invention to provide compositions and methods for stabilizing natriuretic peptides. Such methods may improve the therapeutic potential of natriuretic peptides, particularly for the treatment of cardiovascular diseases. Several natriuretic peptides, including pro-BNP, mature BNP, and pro-ANP comprise a penultimate proline residue, and are suitable substrates for prolyl-specific dipeptidyl peptidases ("DPPs"). Thus, while mature BNP has been reported to exhibit resistance to degradation by neutral endopeptidase relative to ANP, DPPs may represent a previously unrecognized degradation pathway for the mature BNP molecule as well as for pro-BNP and pro-ANP. Furthermore, the removal of the proline-containing dipeptide may open the various natriuretic peptides to further degradation by other peptidases. Subjects that may benefit from increased natriuretic peptide concentrations may be treated with inhibitors of one or more DPPs, either alone or in combination with neutral endopeptidase inhibitors, and/or treated with natriuretic peptides and/or natriuretic peptide analogs exhibiting increased DPP stability. In addition, BNP in samples removed from a subject may be stabilized during storage using these same inhibitors.

Thus, in one aspect, the present invention relates to in vitro methods of inhibiting degradation of one or more natriuretic peptides according to claim 1.

In another aspect, the present invention relates to one or more inhibitors of prolyl-specific dipeptidyl peptidase for use in methods of inhibiting degradation of one or more natriuretic peptides. The method comprises administering one or more inhibitors of prolyl-specific dipeptidyl peptidase in an amount sufficient to inhibit degradation of the natriuretic peptide, wherein said one or more inhibitors of prolyl-specific dipeptidyl peptidase are (1) a dipeptide analogue comprising Xaa-boroPro, wherein Xaa can be any amino acid and boroPro is boronate proline ester, (2) Diprotin A, or (3) antibodies or antibody fragments.

In another aspect, the present invention relates to one or more inhibitors of prolyl-specific dipeptidyl peptidase for use in methods for treating a subject in need of increased natriuretic peptide function, preferably subjects suffering from heart failure, wherein said one or more of said inhibitors of prolyl-specific dipeptidyl peptidase are (1) a dipeptide analogue comprising Xaa-boroPro, wherein Xaa can be any amino acid and boroPro is boronate proline ester, (2) Diprotin A, or (3) antibodies or antibody fragments. The methods comprise administering one or more inhibitors of prolyl-specific dipeptidyl peptidase to the subject, preferably in an amount sufficient to inhibit degradation of the natriuretic peptide.

The inhibitor(s) of prolyl-specific DPP may be selective for one or more DPP(s) for which pro-BNP, mature BNP, and/or pro-ANP are a substrate. Methods for designing and selecting specific DPP inhibitors are well known in the art. *See*, *e.g.*, Leiting et al., Biochem. J. 371: 525-32, 2003; Sedo et al., Physiol. Res. 52: 367-72, 2003; Villhauer et al., J. Med. Chem. 46: 2774-89, 2003; Senten et al., J. Comb. Chem. 5: 336-44, 2003; Senten et al., Bioorg. Med. Chem. Lett. 12: 2825-8, 2002; Borloo and Meester, Verh. K. Acad Geneeskd. Belg. 56: 57-88, 1994. In addition, DPP may be inhibited at the level of expression by methods known to those of skill in the art, such as by antisense or RNAi constructs. DPPs may also be inhibited through the use of binding proteins, *e.g.*, antibodies or fragments thereof that specifically bind to one or more DPPs and prevent their activity on a natriuretic peptide substrate.

The methods described herein may comprise the use of one or more inhibitors of prolyl-specific DPP alone, or such inhibitors may be combined with one or more inhibitors of neutral endopeptidase and/or other protease inhibitors, and/or with one or more exogenously added natriuretic peptides to provide a potentiated increase in natriuretic peptide function to the subject in comparison to the use of inhibitors of neutral Endopeptidase and/or exogenously added natriuretic peptides in the absence of prolyl-specific DPP inhibitor(s). These compounds may be conveniently provided as part of a pharmaceutical composition.

In preferred embodiments, subjects receiving the treatment methods described herein suffer from diseases selected from the group consisting of stroke, congestive heart failure (CHF), cardiac ischemia, systemic hypertension, and/or acute myocardial infarction. In particularly preferred embodiments, subjects receiving the treatment methods described herein are selected on the basis of a BNP level. For example, subjects may be selected on the basis of a plasma BNP level prior to receiving treatment of at least about 80 pg/mL, preferably at least about 100 pg/mL, still more preferably at least about 200 pg/mL, yet more preferably at least about 500 pg/mL, and most preferably at least about 1000 pg/mL.

The above described methods for treating a subject in need of increased natriuretic peptide function can further comprise administering one or more analogues of a natriuretic peptide that provide increased stability in the presence of prolyl-specific DPP (e.g. as measured by an increase in the t_{½} of the natriuretic peptide of interest in the blood of the subject).

Other features and advantages of the invention will be apparent from the following detailed description of the invention, and from the claims.

**BRIEF DESCRIPTION OF THE FIGURES**

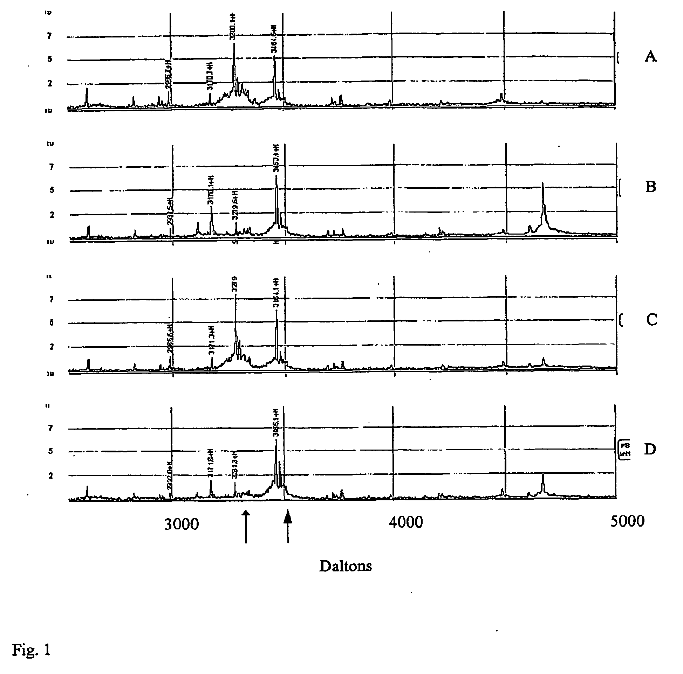

Fig. 1 shows a mass spectrum of BNP and its degradation products in human serum in the absence (panels A and C) and the presence (panels B and D) of an inhibitor of dipeptidyl peptidase.

**DETAILED DESCRIPTION OF THE INVENTION**

The term natriuretic peptide" as used herein refers to members of a group of naturally occurring polypeptide hormones that act in the body to oppose the activity of the renin-angiotensin system, and their biosynthetic precursors and biologically active fragments. There are three major human natriuretic peptides: atrial natriuretic peptide (ANP), which is synthesized in the atria; brain-type natriuretic peptide (BNP), which is synthesized in the ventricles; and C-type natriuretic peptide (CNP), which is synthesized in the brain.

The term "intact natriuretic peptide" as used herein refers to the full length pre-pro-natriuretic peptide, full length pro-natriuretic peptide, full length mature natriuretic peptide, and/or the full length portions removed during processing of the pre-pro- or pro-natriuretic peptides during biosynthesis. In the case of BNP for example, the term "intact natriuretic peptides" encompasses the full length 32 amino acid mature BNP hormone; the full length 134-amino acid pre-pro-BNP molecule; the full length 108-amino acid pro-BNP molecule; the full length 76-amino acid NT-pro BNP molecule, and/or the full length 26-amino acid "pro" peptide.

The sequence of the human 108 amino acid BNP precursor pro-BNP (BNP₁₋₁₀₈) is shown as SEQ ID NO: 1. Mature, full length BNP (BNP₇₇₋₁₀₈) is shown underlined:

HPLGSPGSAS DLETSGLQEQ RNHLQGKLSE LQVEQTSLEP LQESPRPTGV 50
WKSREVATEG IRGHRKMVLY TLRAPRSPKM VQGSGCFGRK MDRISSSSGL 100
GCKVLRRH 108
(SEQ ID NO: 1).

Human BNP₁₋₁₀₈ is synthesized as a larger precursor pre-pro-BNP having the sequence shown as SEQ ID NO: 2 (with the "pre" sequence shown in bold):

MDPQTAPSRA LLLLLFLHLA FLGGRSHPLG SPGSASDLET SGLQEQRNHL 50
QGKLSELQVE QTSLEPLQES PRPTGVWKSR EVATEGIRGH RKMVLYTLRA 100
PRSPKMVQGS GCFGRKMDRI SSSSGLGCKV LRRH 134

(SEQ ID NO: 2).

The sequence of the 126 amino acid human ANP precursor pro-ANP (ANP₁₋₁₂₆) is shown as SEQ ID NO: 3, with mature, full length ANP (ANP₉₉₋₁₂₆) underlined:

NPMYNAVSNA DLMDFKNLLD HLEEKMPLED EVVPPQVLSD PNEEAGAALS 50
PLPEVPPWTG EVSPAQRDGG ALGRGPWDSS DRSALLKSKL RALLTAPRSL 100
RRSSCFGGRM DRIGAQSGLG CNSFRY 126
(SEQ ID NO: 3).

Human ANP₁₋₂₆ is synthesized as a larger precursor pre-pro-ANP having the sequence shown in SEQ ID NO: 4 (with the "pre" sequence shown in bold):

 50
 100
 150
 151
(SEQ ID NO: 4).

The sequence of the 126 amino acid human CNP precursor pro-CNP (CNP₁₋₁₂₆) is shown as SEQ ID NO: 5, with the full length mature CNP form CNP-53 (CNP₇₄₋₁₂₆) shown in italics, and the full length mature CNP form CNP-22 (CNP₁₀₅₋₁₂₆) shown underlined:

MHLSQLLACA LLLTLLSLRP SEAKPGAPPK VPRTPPAEEL AEPQAAGGGQ 50
KKGDKAPGGG GANLKGDRSR LLRDLRVDTK SRAAWARLLQ EHPNARKYKG 100
ANKKGLSKGC FGLKLDRIGS MSGLGC 126

(SEQ ID NO: 5).

The term "fragment" as used herein refers to a polypeptide that comprises at least six contiguous amino acids of a polypeptide from which the fragment is derived, but is less than the complete parent polypeptide. Thus, a fragment of pro-BNP (BNP₁₋₁₀₈) refers to a polypeptide that comprises at least six contiguous amino acids of BNP₁₋₁₀₈; a fragment of mature BNP refers to a polypeptide that comprises at least six contiguous amino acids of BNP₇₇-₁₀₈; a fragment of the polypeptide generated by cleavage ofpro-BNP into mature BNP refers to a polypeptide that comprises at least six contiguous amino acids of BNP₁₋₇₆. Similarly, a fragment of pro-ANP (ANP₁₋₁₂₆) refers to a polypeptide that comprises at least six contiguous amino acids of ANP₁₋₁₂₆; a fragment of mature ANP refers to a polypeptide that comprises at least six contiguous amino acids of ANP₉₉₋₁₂₆; a fragment of the polypeptide generated by cleavage of pro-ANP into mature ANP refers to a polypeptide that comprises at least six contiguous amino acids of BNP₁₋₉₈; and a fragment of pro-CNP (CNP₁₋₁₂₆) refers to a polypeptide that comprises at least six contiguous amino acids of CNP₁₋₁₂₆; a fragment of mature CNP refers to a polypeptide that comprises at least six contiguous amino acids of CNP₇₄₋₁₂₆ or CNP₁₀₅₋₁₂₆; a fragment of the polypeptide generated by cleavage of pro-CNP into mature CNP refers to a polypeptide that comprises at least six contiguous amino acids of CNP₁₋₇₃ or CNP₁₋₁₀₄. In preferred embodiments, a fragment refers to a polypeptide that comprises at least 10 contiguous amino acids of a polypeptide from which the fragment is derived; at least 15 contiguous amino acids of a polypeptide from which the fragment is derived; or at least 20 contiguous amino acids of a polypeptide from which the fragment is derived.

The term "related fragment" as used herein refers to one or more fragments of a particular polypeptide or its biosynthetic parent that may be detected as a surrogate for the polypeptide itself or as independent markers. For example, human BNP is derived by proteolysis of a 108 amino acid precursor molecule, referred to hereinafter as BNP₁₋₁₀₈. Mature BNP, or "the BNP natriuretic peptide," or "BNP-32" is a 32 amino acid molecule representing amino acids 77-108 of this precursor, which may be referred to as BNP₇₇₋₁₀₈. The remaining residues 1-76 are referred to hereinafter as BNP₁₋₇₆. BNP₁₋₁₀₈ and BNP₁₋₇₆ are examples of "BNP-related fragments."

The term "fragment formed by removal of an N-terminal portion" as used herein in reference to natriuretic peptide fragments refers to a fragment of an intact natriuretic peptide formed by removal of one or more amino acids from the amino terminal end of the intact peptide. In preferred embodiments, such a fragment is formed by removal of at least 2, 3, 4, 5, 7,10,15, 20, or more amino acids from the amino terminal end of the intact peptide.

The term "biologically active" as used herein in reference to natriuretic peptides and fragments thereof refers to a full length mature natriuretic peptide; or a polypeptide derived from the full length mature natriuretic peptide or its precursor molecules that exhibit at least 50% of the vasorelaxation effects in isolated preconstricted mouse aortic rings exhibited by the full length mature natriuretic peptide, measured as described in Lopez et al., J. Biol. Chem. 272: 23064-23068, 1997. Biologically active natriuretic peptides may include fragments of the full length mature natriuretic peptide, or precursor forms or fragments thereof.

The term "biologically inactive" as used herein in reference to natriuretic peptide fragments refers to a polypeptide derived from the full length mature natriuretic peptide or its precursor that is not "biologically active" as defined above. As used herein, the term "biologically inactive" does not necessarily refer to a complete loss of all biological activity. Rather, a "biologically inactive" natriuretic peptide fragment preferably exhibits less than 50%, preferably less than 25%, more preferably less than 10%, and most preferably less than 1%, of one or more biological functions of the intact natriuretic peptide. This biological function may be receptor binding, which may be measured as described in Smith et al., J. Endocrinol. 167: 239-46, 2000, cGMP production in cultured rat aortic smooth muscle cells, which may be measured as described in Shimekake et al., FEBS Lett. 309: 185-9,1992, and/or the vasorelaxation effects in isolated preconstricted mouse aortic rings exhibited by the full length mature natriuretic peptide, measured as described in Lopez et al., J. Biol. Chem. 272: 23064-23068, 1997, compared to that exhibited by the full length mature natriuretic peptide.

As used herein, the term "purified" in reference to polypeptides does not require absolute purity. Instead, it represents an indication that the polypeptide(s) of interest is(are) in a discrete environment in which abundance (on a mass basis) relative to other proteins is greater than in a biological sample. By "discrete environment" is meant a single medium, such as a single solution, a single gel, a single precipitate, *etc.* Purified polypeptides may be obtained by a number of methods including, for example, laboratory synthesis, chromatography, preparative electrophoresis, centrifugation, precipitation, affinity purification, etc. One or more "purified" polypeptides of interest are preferably at least 10% of the protein content of the discrete environment. One or more "substantially purified" polypeptides are at least 50% of the protein content of the discrete environment, more preferably at least 75% of the protein content of the discrete environment, and most preferably at least 95% of the protein content of the discrete environment. Protein content is determined using a modification of the method of Lowry et al., J. Biol. Chem. 193: 265, 1951, described by Hartree, Anal Biochem 48: 422-427 (1972), using bovine serum albumin as a protein standard.

The term "antibody" as used herein refers to a peptide or polypeptide derived from, modeled after or substantially encoded by an immunoglobulin gene or immunoglobulin genes, or fragments thereof, capable of specifically binding an antigen or epitope. *See*, *e.g.* Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, *i.e.*, "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies, monoclonal antibodies, polyclonal antibodies, and antibodies obtained by molecular biological techniques (e.g., by phage display methods) are also included by reference in the term "antibody." Preferred antibodies are "Omniclonal" antibodies. By this is meant a mixture of different antibody molecules selected from a phage display library, where each antibody specifically binds to a target antigen with a minimum affinity of 10⁹ M⁻¹ to 10¹⁰ M⁻¹.

The term "specifically binds" is not intended to indicate that an antibody binds exclusively to its intended target. Rather, an antibody "specifically binds" if its affinity for its intended target is about 5-fold greater when compared to its affinity for a non-target molecule. Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Specific binding between an antibody or other binding agent and an antigen means a binding affinity of at least 10⁶ M⁻¹. Preferred antibodies bind with affinities of at least about 16⁷ M⁻¹, and preferably between about 10⁸ M⁻¹ to about 10⁹M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off} /kₒₙ (k_{off} is the dissociation rate constant, kₒₙ is the association rate constant and K_{d} is the equilibrium constant. Affinity can be determined at equilibrium by measuring the fraction bound (r) of labeled ligand at various concentrations (c). The data are graphed using the Scatchard equation: r/c = K(n-r):
where
r = moles of bound ligand/mole of receptor at equilibrium;
c = free ligand concentration at equilibrium;
K = equilibrium association constant; and n = number of ligand binding sites per receptor molecule
By graphical analysis, r/c is plotted on the Y-axis versus r on the X-axis thus producing a Scatchard plot. The affinity is the negative slope of the line. k_{off} can be determined by competing bound labeled ligand with unlabeled excess ligand (see, e.g., U.S. Pat No. 6,316,409). The affinity of a targeting agent for its target molecule is preferably at least about 1 x 10⁻⁶ moles/liter, is more preferably at least about 1 x 10⁻¹ moles/liter, is even more preferably at least about 1x10⁻⁸ moles/liter, is yet even more preferably at least about 1x10⁻⁹ moles/liter, and is most preferably at least about 1 x 10⁻¹⁰ moles/liter. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "discrete" as used herein refers to areas of a surface that are non-contiguous. That is, two areas are discrete from one another if a border that is not part of either area completely surrounds each of the two areas. The term "independently addressable" as used herein refers to discrete areas of a surface from which a specific signal may be obtained. One skilled in the art will appreciate that antibody zones can also be independent of each other, but can be in contact with each other on a surface.

The term "test sample" as used herein refers to a sample in which the presence or amount of one or more analytes of interest are unknown and to be determined in an assay, preferably an immunoassay. Preferably, a test sample is a bodily fluid obtained for the purpose of diagnosis, prognosis, or evaluation of a subject, such as a patient. In certain embodiments, such a sample may be obtained for the purpose of determining the outcome of an ongoing condition or the effect of a treatment regimen on a condition. Preferred test samples include blood, serum, plasma, cerebrospinal fluid, urine and saliva. In addition, one of skill in the art would realize that some test samples would be more readily analyzed following a fractionation or purification procedure, for example, separation of whole blood into serum or plasma components. Preferred samples may be obtained from bacteria, viruses and animals, such as dogs and cats. Particularly preferred samples are obtained from humans. By way of contrast, a "standard sample" refers to a sample in which the presence or amount of one or more analytes of interest are known prior to assay for the one or more analytes

The term "disease sample" as used herein refers to a tissue sample obtained from a subject that has been determined to suffer from a given disease. Methods for clinical diagnosis are well known to those of skill in the art. *See*, *e.g.*, Kelley's Textbook of Internal Medicine, 4th Ed., Lippincott Williams & Wilkins, Philadelphia, PA, 2000; The Merck Manual of Diagnosis and Therapy, 17th Ed., Merck Research Laboratories, Whitehouse Station, N.J., 1999.

The terms "prolyl-specific dipeptidyl peptidase" or "prolyl-specific DPP" refer to serine proteases that cleave dipeptides from the N-terminal of substrate polypeptides, and that exhibit a preference for proline in the second position (i.e., NH2-X-pro-peptide-COOH where X is an amino acid, and the bond between pro and the remaining peptide is cleaved). Such proteases are generally classified under B.C.3.4.14.X, including E.C.3.4.14.5 and 3.4.14.11. DPPs are often classified into types such as DPP-II and DPP-IV.

The term "inhibitor" as used herein in reference to molecules that affect an enzymatic (e.g., proteolytic) activity does not necessarily refer to a complete loss of all enzymatic activity. Rather, an "inhibitor" reduces an enzymatic activity by at least 10%, more preferably at least 25%, even more preferably by at least 50%, still more preferably by at least 75%, and most preferably by at least 90%, of the enzymatic activity exhibited in the absence of the inhibitor. *In vitro,* the activity of an inhibitor may be measured by directly measuring enzymatic activity by methods well known to those of skill in the art. *In vivo*, the activity of an inhibitor may also be measured by directly measuring enzymatic activity on the enzyme substrate, or in the case of a degradative enzyme, may be measured by determining a time (T_{½}) in which ½ of the substrate is cleared from the body of a subject (e.g., an experimental animal). In the latter case, an "inhibitor" increases a T_{½} by at least 10%, more preferably at least 25%, even more preferably by at least 50%, still more preferably by at least 75%, and most preferably by at least 90%, compared to the T_{½} exhibited in the absence of the inhibitor.

Preferred inhibitors are selective for a particular class of proteases (e.g., selective for dipeptidyl peptidase or for a particular subset of dipeptidyl peptidase). An inhibitor is said to be "selective" for a particular class of protease if it inhibits that class at least 10-fold more, more preferably at least. 100-fold more, and most preferably at least 1000-fold more, than non-target proteases. Selective inhibitors of various DPP types are known. For example, H-Dab-Pip is reportedly selective (> 7,600-fold) for dipeptidyl peptidase II (DPP II; EC 3.4.14.2) over DPP IV (IC₅₀ > 1 mM) (DPP IV; EC 3.4.14.5). Senten et al., Bioorg. Med. Chem. Lett. 12: 2825, 2002. Similarly, 1-[[[2-[(5-cyanopyzidin-2-yl)amino]ethyl]amino]acetyl]-2-cyano-(S)-pyrrolidine is reportedly a selective, orally active inhibitor of DPP IV. Ahren et al., Diabetes Care 25:869-75, 2002.

The term "about" as used herein refers to +/- 10% of a given number.

Use of natriuretic peptide fragments as prognostic and diagnostic markers

As noted above, increased blood levels of natriuretic peptides have been found in certain disease states, suggesting a role in the pathophysiology of those diseases, including stroke, congestive heart failure (CHF), cardiac ischemia, systemic hypertension, and acute myocardial infarction. *See*, *e.g.*, WO 02/089657; WO 02/083913; WO 03/016910; Hunt et al., Biochem. Biophys. Res. Comm. 214: 1175-83 (1995); Venugopal, J. Clin. Pharm. Ther. 26: 15-31, 2001; and Kalra et al., Circulation 107: 571-3,2003. The natriuretic peptides, alone, collectively, and/or together with additional proteins, can also serve as disease markers and indicators of prognosis in various cardiovascular conditions.

It has been reported that removal of natriuretic peptides from the circulation involves degradation pathways. Indeed, inhibitors of neutral Endopeptidase, which cleaves natriuretic peptides under certain circumstances, have been suggested to hold promise in treatment of certain cardiovascular diseases. *See, e.g.,* Trindade and Rouleau, Heart Fail. Monit. 2: 2-7, 2001. However, the measurement of the natriuretic peptides in clinical samples has focused generally upon measurement of BNP, ANP, and/or CNP; their precursor molecules (i.e., pro-BNP, pro-ANP, and pro-CNP); and the fragments resulting from cleavage of the pro-form to provide the mature natriuretic peptides, without consideration of the degradation state of the molecules. It has also been reported that oxidation of methionine residues in the natriuretic peptides reduces the biological activity compared to reduced forms. Koyama et al., Eur. J. Biochem. 203: 425-32. For the purposes described herein, the methionine-oxidized forms may be considered products of degradation.

**Assay Measurement Strategies**

Numerous methods and devices are well known to the skilled artisan for the detection and analysis of polypeptides or proteins in test samples. In preferred embodiments, immunoassay devices and methods are often used. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. *See, e.g.,* U.S. Patents 5,631,171; and S,955,377. One skilled in the art also recognizes that robotic instrumentation including but not limited to Beckman Access, Abbott AxSym, Roche ElecSys, Dade Behring Stratus systems are among the immunoassay analyzers that are capable of performing the immunoassays taught herein. Specific immunological binding of the antibody to the marker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like.

The use of immobilized antibodies specific for the one or more polypeptides is also described herein. The antibodies could be immobilized onto a variety of solid supports, such as magnetic or chromatographic matrix particles, the surface of an assay place (such as microtiter wells), pieces of a solid substrate material or membrane (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a colored spot.

The analysis of a plurality of polypeptides may be carried out separately or simultaneously with one test sample. For separate or sequential assay, suitable apparatuses include clinical laboratory analyzers such as the ElecSys (Roche), the AxSym (Abbott), the Access (Beckman), the ADVIA® CENTAUR® (Bayer) immunoassay systems, the NICHOLS ADVANTAGE® (Nichols Institute) immunoassay system, etc. Preferred apparatuses or protein chips perform simultaneous assays of a plurality of polypeptides on a single surface. Particularly useful physical formats comprise surfaces having a plurality of discrete, adressable locations for the detection of a plurality of different analytes. Such formats include protein microarrays, or "protein chips" (*see, e.g.,* Ng and Ilag, J. Cell Mol. Med. 6: 329-340 (2002)) and certain capillary devices (*see, e.g.,* U.S. Patent No. 6,019,944). In these embodiments, each discrete surface location may comprise antibodies to immobilize one or more analyte(s) (e.g., one or more polypeptides of the invention) for detection at each location. Surfaces may alternatively comprise one or more discrete particles (e.g., microparticles or nanoparticles) immobilized at discrete locations of a surface, where the microparticles comprise antibodies to immobilize one analyte (e.g., one or more polypeptides , of the invention) for detection.

In addition, one skilled in the art would recognize the value of testing multiple samples (for example, at successive time points) from the same individual. Such testing of serial samples will allow the identification of changes in polypeptide levels over time. Increases or decreases in polypeptide levels, as well as the absence of change in such levels, would provide useful information about the disease status that includes, but is not limited to identifying the approximate time from onset of the event, the presence and amount of salvagable tissue, the appropriateness of drug therapies, the effectiveness of various therapies as indicated by reperfusion or resolution of symptoms, differentiation of the various types of disease having similar symptoms, identification of the severity of the event, identification of the disease severity, and identification of the patient's outcome, including risk of future events.

A panel consisting of the polypeptides referenced above, and optionally including other protein markers useful in diagnosis, prognosis, or differentiation of disease, may be constructed to provide relevant information related to differential diagnosis. Such a panel may be constructed to detect 1, 2, 3, 4, 5, 6, 7, 8, 9, 10, 15, 20, or more or individual analytes, including one or more polypeptides described herein. The analysis of a single analyte or subsets of analytes could be carried out by one skilled in the art to optimize clinical sensitivity or specificity in various clinical settings. These include, but are not limited to ambulatory, urgent care, critical care, intensive care, monitoring unit, inpatient, outpatient, physician office, medical clinic, and health screening settings. Furthermore, one skilled in the art can use a single analyte or a subset of analytes in combination with an adjustment of the diagnostic threshold in each of the aforementioned settings to optimize clinical sensitivity and specificity. The clinical sensitivity of an assay is defined as the percentage of those with the disease that the assay correctly predicts, and the specificity of an assay is defined as the percentage of those without the disease that the assay correctly predicts (Tietz Textbook of Clinical Chemistry, 2nd edition, Carl Burtis and Edward Ashwood eds., W.B. Saunders and Company, p. 496).

The analysis of analytes could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings.

As discussed above, samples may continue to degrade the natriuretic peptides or fragments thereof, even once the sample is obtained. Thus, it may be advantageous to add one or more protease inhibitors to samples prior to assay. Numerous protease inhibitors are known to those of skill in the art, and exemplary inhibitors may be found in, *e.g.*, The Complete Guide for Protease Inhibition, Roche Molecular Biochemicals, updated June 3, 1999 at http://www.roche-applied-science.com/fst/products.htm?/prod_inf/manuals/protease/prot_toc.htm, and European Patent Application 03013792.1 (published as EP 1 378 242 A1). Because various metalloproteases and calcium-dependent proteases are known to exist in blood-derived samples, chelators such as EGTA and/or EDTA, also act as protease inhibitors. In addition, or in the alternative, inhibitors of neutral endopeptidase and/or DPPs may be used.

Inhibition of Natriuretic Peptide Degradation by Prolyl-Specific DPPs

The neurohumoral regulatory system of which natriuretic peptides are a part represents a complex system of cardiovascular regulation. Diseases such as congestive heart failure are, in essence, fatal diseases for which life may be prolonged, but the underlying disease never cured. Thus, there remains a need for novel therapeutic approaches to the management of the underlying diseases, and multiple points in this complex system are seen as important targets by clinicians. The clinical success of angiotensin converting enzyme ("ACE") inhibitors in disease management has led to a search for additional approaches that indirectly affect the course of cardiovascular disease by affecting enzymes that act on vasoactive hormones.

In the case of the natriuretic hormones, increasing hormone levels have been found to have therapeutic potential in patients. *See, e.g.,* Tsekoura et al., Hellenic J. Cardiol. 44: 266-70, 2003. Neutral endopeptidase ("NEP") is believed to be a key degradation mediator. Not surprisingly, inhibitors of NEP have found use in treating patients with diseases such as hypertension, atherosclerosis, and heart failure. *See, e.g.,* Corti et al., Circulation 104: 1856-62, 2001. Combination treatment with both BNP and NEP inhibitors has been reported to produce a synergistic effect on cardiac output, reduced vascular resistance, and unloading of the heart. Chen et al., Circulation 105: 999-1003, 2002. Targeting NEP may suffer from the limitation, however, that NEP metabolizes a broad range of biologically active peptides. *See*, *e.g.,* Walter et al., Curr. Opin. Nephrol. Hypertens. 6: 468-73, 1997.

The present invention describes a novel approach to treatment of cardiovascular disease, particularly heart failure. Several natriuretic peptides, including human forms of pro-BNP, mature BNP, and pro-ANP comprise a penultimate proline residue, rendering the peptides suitable substrates for prolyl-specific dipeptidyl dipeptidases ("DPPs"). Inhibitors of DPP have been described as having utility in the management of diabetes, mediated by the inhibition of glucose-dependent insulinotropic polypeptide degradation by DPP IV. *See, e.g.,* Gault et al., Biochem. Biophys. Res. Commun. 22: 207-13, 2003. However, their use in treatment of cardiovascular disease has not previously been reported.

Methods for preparing and identifying selective DPP inhibitors are well known in the art. DPP inhibitors include the dipeptide analogues Xaa-boroPro, including Pro-boroPro, Ala-boroPro, Val-boroPro, and Lys-boroPro, and dab-pip. *See, e.g.,* Senten et al., Bioorg. Med. Chem Lett. 12: 2825-28, 2002; Jones et al., Blood, prepublished online May 8, 2003; DOI 10.1182. Combinatorial chemistry methods have been used to rapidly synthesize and screen numerous additional dipeptide analogue inhibitors of DPP. *See, e.g.,* Leiting et al., Biochem. J. 371: 525-32, 2003; Sedo et al., Physiol. Res. 52: 367-72, 2003; Villhauer et al., J. Med. Chem. 46: 2774-89, 2003; Senten et al., J. Comb. Chem. 5: 336-44,2003; and U.S. Patents 5,602,102; 6,573,287, 6,548,481, 6,432,969, and 6,355,614. The compounds described in these publications may be used as lead compounds in identifying additional DPP inhibitors for use in the methods described herein. A variety of techniques are available in the art for generating combinatorial libraries of small organic molecules. *See generally* Blondelle et al. Trends Anal. Chem. 14: 83, 1995; U.S. Patents 5,359,115, 5,362,899, 5,288,514, and 5,721,099; Chen et al. JACS 116: 2661, 1994; Kerr et al. JACS 115: 252, 1993; WO92/10092, WO93/09668, WO 94/08051, WO93/20242 and WO91/07087. A variety of libraries on the order of about 16 to 1,000,000 or more diversomers can be synthesized and screened for a particular activity or property using the methods described therein.

Preferably, the inhibitors finding use in the invention are small molecules, meaning having a molecular weight of less than about 1000 Daltons. Such inhibitors are well known in the art. *See, e.g.,* WO04/07468 and WO04/50022, and U.S. Patents 6,710,040; 6,699,871; 6,432,969; 6,303,661; 6,166,063; 6,124,305; 6,110,949; and 6,107,317. Preferred small molecule inhibitors are orally effective. DPP-inhibitory antibody or antibody fragments may also find use in the methods described herein. In this case, antibodies may be generated to DPP and screened (e.g., using the phage display methods described herein) to identify antibodies that inhibits DPP activity on one or more natriuretic peptides of interest.

Compounds may be screened for inhibitory activity using isolated DPP enzymes, cell extracts, or blood derived samples as a source of enzyme, and isolated natriuretic peptides as substrates. Selection of the conditions to inhibit loss of the penultimate proline residue from a target natriuretic peptide may depend on the type of aqueous medium under consideration (for example, inhibition in a blood sample may require conditions that differ from inhibition in the circulation of an organism). Selecting such conditions are within the skill of the artisan. The ability of test compounds and their corresponding pharmaceutically acceptable acid addition salts to inhibit DPP may also be demonstrated by employing a modified version of the assay described in Kubota et al., Clin. Exp. Immunol. 89: 192-7, 1992. Confirmation of the presence or absence of the penultimate proline residue may be performed using an immunoassay selected to be sensitive to the loss of this N-terminal portion of the molecule, or through the use of mass spectrometry.

Proceeding to the next step, candidate compounds that modulate DPP activity in cultured cells can be tested in animal models that are relevant to the disease condition of interest. In these methods, labeled natriuretic peptide may be injected into a test animal, and the T_{½} for clearance of the natriuretic peptide from the circulation may be determined in the presence and absence of the inhibitors. Preferred animal models of DPP-dependent natriuretic peptide degradation include rats, mice, sheep, dogs, cats, and pigs.

As discussed above, combination treatment with DPP inhibitors and NEP inhibitors and/or natriuretic peptide(s) is disclosed herein. In addition or as an alternative, a natriuretic peptide may be provided as an analogue that has been stabilized to DPP activity, as described for glucose-dependent insulinotropic polypeptide in Gault et al., Metabolism 52: 679-87, 2003. In preferred embodiments, libraries of natriuretic peptide analogs having one or more substituted, deleted, added, or modified amino acids may be screened for improved stability to DPP degradation. Such analogs preferably retain 50% or more of the natriuretic activity of the parent natriuretic peptide.

When administered, the pharmaceutical preparations of the invention are applied in pharmaceutically-acceptable amounts and in pharmaceutically-acceptably compositions. Such preparations may routinely contain salt, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts. The pharmaceutical compositions also may contain, optionally, suitable preservatives, such as: benzalkonium chloride; chlorobutanol; parabens and thimerosal. Carrier formulation suitable for oral, subcutaneous, intravenous, intramuscular, etc. administrations can be found in Remington's Pharmaceutical Sciences, Mack Publishing Co., Easton, Pa.

A variety of administration routes are available for treating a subject. The particular mode of delivery selected will depend upon the particular compound selected, the severity of the condition being treated and the dosage required for therapeutic efficacy. The methods disclosed herein, generally speaking, may be practiced using any mode of administration that is medically acceptable, meaning any mode that produces effective levels of the active compounds without causing clinically unacceptable adverse effects. Such modes of administration include oral, rectal, topical, nasal, interdermal, intravenous or parenteral routes. Such modes of administration also include obtaining T cells or bone marrow cells, stem cells or early lineage progenitor cells from a patient and contacting the isolated cells with the compounds of the invention ex vivo, followed by reintroducing the treated cells to the patient. The treated cells can be reintroduced to the patient in any manner known in the art for administering viable cells.

Oral administration is particularly preferred. Compositions suitable for oral administration may be presented as discrete units, such as capsules, tablets, lozenges, each containing a predetermined amount of the compound of the invention. Other compositions include suspensions in aqueous liquids or non-aqueous liquids such as a syrup, elixir or an emulsion. Preferably, the oral preparation does not include an enteric coating since it is desirable to expose the cyclic compounds of the invention to the acidic pH conditions of the digestive tract to convert the cyclic molecules to their linear counterparts.

Other delivery systems can include time-release, delayed release or sustained release delivery systems. Such systems can avoid repeated administrations of the compounds described above, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di- and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which the compound is contained in a form within a matrix such as those described in U.S. Pat. Nos. 4,452,775, 4,667,014,4,748,034 and 5,239,660 and (b) diffusional systems in which an active component permeates at a controlled rate from a polymer such as described in U.S. Pat. Nos. 3,832,253, and 3,854,480. In addition, pump-based hardware delivery systems can be used, some of which are adapted for implantation.

Use of a long-term sustained release implant may be particularly suitable for treatment of chronic conditions. Long-term release, as used herein, means that the implant is constructed and arranged to delivery therapeutic levels of the active ingredient for at least 10 days, and preferably 60 days. Long-term sustained release implants are well-known to those of ordinary skill in the art and include some of the release systems described above.

The selected compounds are administered in effective amounts. An effective amount is a dosage of the compound sufficient to provide a medically desirable result. The effective amount will vary with the particular condition being treated, the age and physical condition of the subject being treated, the severity of the condition, the duration of the treatment, the nature of the concurrent therapy (if any), the specific route of administration and like factors within the knowledge and expertise of the health practitioner. Generally, doses of active compounds will be from about 0.001 mg/kg per day to 1000 mg/kg per day. It is expected that doses range of 0.001 to 100 mg/kg will be suitable, preferably orally and in one or several administrations per day. Lower doses will result from other forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day are contemplated to achieve appropriate systemic levels of compounds.

Examples

The following examples serve to illustrate the present invention. These examples are in no way intended to limit the scope of the invention.

Example 1: Synthesis of DPP inhibitors

Peptide coupling chemistry is preferably employed to prepare linear boroPro compounds. The peptide coupling chemistry methods and procedures used in this invention are readily available. Examples of books using these methods include, but are not limited to, the following citations P. D. Bailey, "An Introduction to Peptide Chemistry," John Wiley & Sons, 1990; Miklos Bodansky, "Peptide Chemistry A Practical Textbook," Springer-Verlag, 1988; Miklos Bodansky, "Principles of Peptide Synthesis, Reactivity and Structure Concepts in Organic Chemistry," Volume 16, Springer-Verlag, 1984; and Miklos Bodansky, "Principles of Peptide Synthesis, Reactivity and Structure Concepts in Organic Chemistry," Volume 21, Springer-Verlag, 1984.

The compounds of the invention can begin with the synthesis of H-boroPro as disclosed in WO 98/00439. Use of H-boroPro is for illustrative purposes only, and is not intended to limit the scope of this invention. According to WO 98/00439, H-boroPro may be prepared by the synthetic route previously developed and described (G. R. Flentke, et al., "Inhibition of dipeptidyl aminopeptidase IV (DP-IV) by Xaa-boroPro dipeptides and use of these inhibitors to examine the role of DP-IV in T-cell function," PNAS (U.S.A.) 88, 1556-1559 (1991); also described in U.S. Pat. No. 5,462,928). Alternatively, H-boroPro may be produced by a new procedure (Kelly, T. A., et al., "The efficient synthesis and simple resolution of a proline boronate ester suitable for enzyme inhibition studies," Tetrahedron 49, 1009-1016 (1993)). Both of these synthetic routes reportedly yield racemic H-boroPro pinanediol.

According to WO 98/00439, stereochemically pure L, L and L, D diastereomers of Z-Lys-boroPro are prepared by first resolving racemic H-boroPro through crystallization with optically active blocking protecting groups ((1S, 2S, 3R, 5S)-+-pinanediol isomer) followed by coupling the isotopically pure L-boroPro and D-boroPro to the stereochemically pure L isomer of lysine (See U.S. Pat. No. 5,462,928). Alternatively, the L,L and L,D diastereomers of Lys-boroPro are prepared in high optical purity by coupling racemic H-boroPro by L-Lys and separating the resulting diastereomeric Z-Lys-boroPro-diester into its component L,D and L,L diastereomers using reverse phase HPLC as previously described for diastereomeric Pro-boroPro (W. G. Gutheil and W. W. Bachovchin, "Separation of L-Pro-DL-boroPro into Its Component Diastereomers and Kinetic Analysis of Their Inhibition of Dipeptidyl Peptidase IV. A New Method for the Analysis of Slow, Tight-Binding Inhibition," Biochemistry 32, 8723-8731 (1993)).

Example 2: Purification of Dipeptidyl Peptidase

The following examples are exemplary for dipeptidyl peptidase IV; dipeptidyl peptidase II may be isolated and used similarly according to the methods of U.S. Patent 6,485,955.

Porcine enzyme is purified as previously described (1), with several modifications. Kidneys from 15-20 animals are obtained, and the cortex dissected away and frozen at -80 °C. Frozen tissue (2000-2500 g) is homogenized in 12 L of 0.25 M sucrose in a Waring blender. The homogenate is left at 37 °C for 18 hours to facilitate cleavage of DPP4 from cell membranes. After the cleavage step, the homogenate is clarified by centrifugation at 7000xg for 20 minutes at 4 °C, and the supernatant is collected. Solid ammonium sulfate is added to 60% saturation, and the precipitate is collected by centrifugation at 10,000xg and discarded. Additional ammonium sulfate is added to the supernatant to 80% saturation, and the 80% pellet is collected and dissolved in 20 mM Na₂HPO₄, pH 7.4.

After dialysis against 20 mM Na₂HPO₄, pH 7.4, the preparation is clarified by centrifugation at 10,000xg. The clarified preparation then is applied to 300 ml of ConA Sepharose equilibrated in the same buffer. After washing with buffer to a constant A280, the column is eluted with 5% (wt/vol) methyl α-D-mannopyranoside. Active fractions are pooled, concentrated, and dialyzed against 5 mM sodium acetate, pH 5.0. Dialyzed material is flowed through a 100 ml Pharmacia Resource S column equilibrated in the same buffer. The flowthrough material is collected and contained most of the enzyme activity. Active material again is concentrated and dialyzed into 20 mM Na₂HPO₄, pH 7.4. Lastly, the concentrated enzyme is chromatographed on a Pharmacia S-200 gel filtration column to removed low molecular weight contaminants. Purity of column fractions is analyzed by reducing SDS-PAGE, and the purest fractions pooled and concentrated. Purified enzyme is stored in 20% glycerol at -80 °C.

Example 3. Assay of Dipeptidyl Peptidase

Enzyme is assayed under steady-state conditions as previously described in Nagatsu et al., Anal. Biochem. 74: 466-76, 1976 with BNP as substrate, with the following modifications. Reactions contain, in a final volume of 100 µL, 100 mM ACES, 52 mM TRIS, 52 mM ethanolamine, 500 µM substrate, 0.2% DMSO, and 4.5 nM enzyme at 25 °C, pH 7.4. For analysis of positive compounds, steady-state kinetic inhibition constants are determined as a function of both substrate and inhibitor concentration. Complete inhibition experiments contain 11 substrate and 7 inhibitor concentrations, with triplicate determinations. For tight binding inhibitors with Kᵢ s less than 20 nM, the enzyme concentration is reduced to 0.5 nM and reaction times are increased to 120 minutes. Pooled datasets from the three plates are fitted to the appropriate equation for either competitive, noncompetitive or uncompetitive inhibition.

Example 4. Analysis of Natriuretic Peptides by Mass Spectrometry

Preparation of antibody capture surface

3 µL of antibody solution (0.25 mg/mL anti-BNP monoclonal antibody in borate buffered saline pH 8.0 ("BBS")) is applied to appropriate spots of a PS10 ProteinChip array (Ciphergen cat. # C553-0044), and the chip is placed in a humid chamber with gentle agitation at 20 °C for 3 hours. The antibody solution is removed, and the array spots are washed twice with 3 µL of 1.5 mg/mL BSA/0.1% Triton X-10010.5 M Tris-HCl pH 8.0. At the second wash, the chip is placed in a humid chamber without agitation at 20 °C for 3 hours. Following this wash, the array is immersed in 5 mM HEPES pH 7.5, and the excess buffer is removed.

Capture of BNP

Using a BIOMEC robotic pipetting station (Beckman Instruments), the array is washed with 150 µL 1% Triton X-100 in BBS for 10 minutes; 150 µL 10% PEG300/0.1% Triton X-100 in BBS for 10 minutes; and 3x with 150 µL 0.2% Triton X-100 in BBS for 5 minutes each. 40 µL 0.2% Triton X-100 in BBS and 40 µL deglycosylated sample (or control sample) is applied and incubated overnight at 4 °C with gentle agitation.

Application of energy absorbing matrix and MS analysis

Following this incubation, the array is washed 3x with 150 µL 1M urea/0.1% CHAPS/0.3M KCl/50mM Tris-HCl pH 7.5 for 1 minute each; and 3x with 300 µL 5 mM HEPES pH 7.5 for 3 seconds each. Excess buffer is removed, and the array is allowed to air dry until no sheen is visible. For low molecular weight analysis (M/Z < 6000), 1 µL of 20% α-cyano-4-hydroxycinnamic acid (CHCA, Ciphergen cat. # C300-0001) in 0.5% trifluoroacetic acid (Pierce cat # 28904)/50% acetonitrile (Pierce cat. # 20062) is applied to appropriate spots as an energy absorbing matrix ("EAM"). For high molecular weight analysis (M/Z ≥6000), 1 µL of 50% sinapinic acid (SPA, Ciphergen Cat. No. C300-0002) in 0.5% trifluoroacetic acid/50% acetonitrile is applied to appropriate spots as an EAM. Spots are allowed to air dry, and a second 1 µL drop of the appropriate EAM is applied.

MS spectra are acquired using a Ciphergen ProteinChip reader model PBS IIC. For low molecular weight analysis, the following instrument parameters are used: high mass is set to 70 kDa optimized from 2 kDa to 15kDa, starting laser intensity is set to 165; starting detector sensitivity is set to 9; mass deflector is set to 1 kDa; acquisition method is set to SELDI quantitation; SELDI acquisition parameters=26, delta=10, transients per=18, ending position=76; and warming positions with 5 shots at intensity=175. For high molecular weight analysis, the following instrument parameters are used: high mass is set to 70 kDa optimized from 3 kDa to 30 kDa; starting laser intensity is set to 200; starting detector sensitivity is set to 9; mass deflector is set to 2 kDa; acquisition method is set to SELDI quantitation; SELDI acquisition parameters=24, delta=10, transients per=13, ending position=74; and warming positions with 3 shots at intensity=210.

Example 5. Inhibition of BNP Degradation

Two human plasma samples were individually divided into two tubes, to one of which the reversible DPP inhibitor Diprotin A (Ile-Pro-Ile) was added to a concentration of 1 mM. Each sample was spiked with human BNP₇₇₋₁₀₈, and the mixture held at 4°C overnight. Each sample was subjected to antibody capture SELDI mass spectrometry as described above. The results are depicted in Fig. 1. Panels A (no addition) and B (Diprotin A) represent one plasma sample, and panels C (no addition) and D (Diprotin A) the second plasma sample. Capture was performed with an antibody that recognizes human BNP.

Full length BNP₇₇₋₁₀₈ would be expected to appear at a molecular weight of about 3466 Da (large arrowhead), and BNP₇₉₋₁₀₈ (in which cleavage occurs following the penultimate proline) at about 3282 Da (small arrowhead). Comparing panels A to B and C to D, the cleavage of BNP₇₇₋₁₀₈ to BNP₇₉₋₁₀₈ is inhibited by the DPP inhibitor. A second cleavage product, presumed to be BNP₇₉₋₁₀₆ at a molecular weight of about 2988 Da, is also inhibited by the Diprotin A treatment, with a corresponding increase in a cleavage product presumed to be BNP₇₇₋₁₀₆ at a molecular weight of about 3173 Da. Thus, removal of the amino terminal ser-pro dipeptide is sensitive to the presence of a DPP inhibitor, while a second carboxyl terminal dipeptide cleavage is not.

## Claims

1. An *in vitro* method of inhibiting degradation of one or more natriuretic peptides comprising an amino terminal penultimate proline residue in an aqueous medium, comprising:
contacting said aqueous medium with one or more inhibitors of a prolyl-specific dipeptidyl peptidase under conditions selected to inhibit loss of said proline residue from one or more of said natriuretic peptides present in said sample.

2. A method according to claim 1, wherein said contacting step inhibits loss of an amino terminal penultimate proline residue from BNP.

3. A method according to claim 1 or 2, wherein one or more of said inhibitors of prolyl-specific dipeptidyl peptidase are small molecule inhibitors, or wherein one or more of said inhibitors of prolyl-specific dipeptidyl peptidase comprise antibodies or antibody fragments.

4. The method according to claim 3, wherein the one or more inhibitors of prolyl-specific dipeptidyl peptidase comprise a dipeptide analogue comprising Xaa-boroPro, wherein Xaa can be any amino acid and boroPro is boronate proline ester.

5. The method according to claim 3, wherein the one or more inhibitors of prolyl-specific dipeptidyl peptidase comprise Diprotin A

6. A method according to any of claims 1 to 5, wherein said aqueous medium is blood, serum, or plasma.

7. A method according to any of claims 1 to 6, further comprising performing an assay for one or more natriuretic peptides present on all or a portion of said aqueous medium.

8. Use of one or more inhibitors of prolyl-specific dipeptidyl peptidase for the manufacture of a medicament for treating a subject in need of increased natriuretic peptide function, wherein said one or more of said inhibitors of prolyl-specific dipeptidyl peptidase are (1) a dipeptide analogue comprising Xaa-boroPro, wherein Xaa can be any amino acid and boroPro is boronate proline ester, (2) Diprotin A, or (3) antibodies or antibody fragments.

9. Use of one or more inhibitors of prolyl-specific dipeptidyl peptidase for the manufacture of a medicament for inhibiting degradation of a natriuretic peptide present in a subject, comprising administering said one or more inhibitors of prolyl-specific dipeptidyl peptidase in an amount sufficient to inhibit degradation of the natriuretic peptide by prolyl-specific dipeptidyl peptidase, wherein said one or more of said inhibitors of prolyl-specific dipeptidyl peptidase are (1) a dipeptide analogue comprising Xaa-boroPro, wherein Xaa can be any amino acid and boroPro is boronate proline ester, (2) Diprotin A, or (3) antibodies or antibody fragments.

10. Use according to claim 9, further comprising:
determining the presence or amount of one or more natriuretic peptides in a sample obtained from said subject; and
determining a treatment regimen based on the presence or amount of said one or more natriuretic peptides.

11. The use according to any of claims 8 to 10, wherein said subject is selected to receive said one or more inhibitors of prolyl-specific dipeptidyl peptidase based on a diagnosis selected from the group consisting of stroke, congestive heart failure, cardiac ischemia, systemic hypertension, and acute myocardial infarction.

12. The use according to any of claims 8 to 11, wherein said subject is selected to receive said one or more inhibitors of prolyl-specific dipeptidyl peptidase based on having a plasma BNP level of at least 80 pg/ml prior to administering the medicament.

13. The use according to any of claims 8 to 12, wherein said use further comprises administering BNP or an analog thereof to said subject.

14. The use according to any of claims 8 to 13, wherein said use further comprises administering an inhibitor of neutral endopeptidase to said subject or, wherein said use further comprises administering BNP or an analog thereof and an inhibitor of neutral endopeptidase to said subject.

15. A pharmaceutical composition comprising one or more inhibitors of prolyl-specific dipeptidyl peptidase and a natriuretic peptide.

## Patentansprüche

1. Ein *in vitro*-Verfahren zur Inhibition des Abbaus in einem wässrigen Medium von einem oder mehreren natriuretischen Peptiden, umfassend einen amino-terminalen vorletzten Prolinrest, wobei das Verfahren umfasst:
In-Kontakt-Bringen des wässrigen Mediums mit einem oder mehreren Inhibitoren einer Prolyl-spezifischen Dipeptidylpeptidase unter Bedingungen, die ausgewählt werden, um den Verlust dieses Prolinrestes von dem einem oder mehreren in der Probe vorhandenen natriuretischen Peptiden zu inhibieren.

2. Ein Verfahren nach Anspruch 1, wobei der Schritt des In-Kontakt-Bringens den Verlust eines amino-terminalen vorletzten Prolinrestes aus BNP umfasst.

3. Ein Verfahren nach Anspruch 1 oder 2, wobei einer oder mehrere dieser Inhibitoren der Prolyl-spezifischen Dipeptidylpeptidase kleine-Molekül-Inhibitoren sind, oder wobei einer oder mehrere dieser Inhibitoren der Prolyl-spezifischen Dipeptidylpeptidase Antikörper oder Antikörperfragmente umfasst.

4. Das Verfahren nach Anspruch 3, wobei der einer oder mehrere Inhibitoren einer Prolyl-spezifischen Dipeptidylpeptidase ein Dipeptid-Analogon umfasst, das Xaa-boroPro umfasst, wobei Xaa jede beliebige Aminosäure sein kann und boroPro ein Boronate-Prolinester ist.

5. Das Verfahren nach Anspruch 3, wobei der einer oder mehrere Inhibitoren einer Prolyl-spezifischen Dipeptidylpeptidase Diprotin A umfasst.

6. Ein Verfahren nach einem der Ansprüche 1 bis 5, wobei das wässrige Medium Blut, Plasma oder Serum ist.

7. Ein Verfahren nach einem der Ansprüche 1 bis 6, das weiterhin umfasst, einen Test auf eines oder mehrere vorhandene natriuretische Peptide in einem Teil oder den gesamten wässrigen Mediums durchzuführen.

8. Verwendung von einem oder mehreren Inhibitoren einer Prolyl-spezifischen Dipeptidylpeptidase zur Herstellug eines Medikaments zur Behandlung eines Individuums, das eine erhöhte natriuretische Peptidfunktion benötigt, wobei der eine oder mehrere dieser Inhibitoren einer Prolyl-spezifischen Dipeptidylpeptidase sind (1) ein Dipeptid-Analogon, das Xaa-boroPro umfasst, wobei Xaa jede beliebige Aminosäure sein kann und boroPro ein Boronate-Prolinester ist, (2) Diprotin A oder (3) Antikörper oder Antikörperfragmente.

9. Verwendung von einem oder mehreren Inhibitoren einer Prolyl-spezifischen Dipeptidylpeptidase zur Herstellug eines Medikaments zur Inhibition des Abbaus von einem in einem Individuum vorhandenen natriuretischen Peptid, umfassend Verabreichen des einen oder der mehreren Inhibitoren einer Prolyl-spezifischen Dipeptidylpeptidase in einer Menge, die ausreichend ist, den Abbau des natriuretischen Peptides zu inhibieren, wobei der eine oder mehrere dieser Inhibitoren einer Prolyl-spezifischen Dipeptidylpeptidase sind (1) ein Dipeptid-Analogon, das Xaa-boroPro umfasst, wobei Xaa jede beliebige Aminosäure sein kann und boroPro ein Boronate-Prolinester ist, (2) Diprotin A oder (3) Antikörper oder Antikörperfragmente.

10. Verwendung nach Anspruch 9, weiterhin umfassend:
Bestimmen der Gegenwart oder Menge eines oder mehrerer natriuretischer Peptide in einer von dem Individuum erhaltenen Probe,
Bestimmen einer Behandlungsschemas basierend auf der Gegenwart oder Menge des eines oder der mehreren natriuretischer Peptide.

11. Die Verwendung nach einem der Ansprüche 8 bis 10, wobei das Individuum ausgewählt wird, um den einen oder der mehreren Inhibitoren der Prolyl-spezifischen Dipeptidylpeptidase basierend auf einer Diagnose zu erhalten, die aus der Gruppe ausgewählt ist, die aus Schlaganfall, kongestiver Herzinsuffizienz, Herzischämie, systemischem Bluthochdruck und akuten Myokardinfarkt besteht.

12. Die Verwendung nach einem der Ansprüche 8 bis 11, wobei das Individuum ausgewählt wird, um den einen oder der mehreren Inhibitoren der Prolyl-spezifischen Dipeptidylpeptidase basierend auf einem Plasmaspiegel von BNP von zumindest 80pg/ml vor dem Verabreichen des Medikaments zu erhalten.

13. Die Verwendung nach einem der Ansprüche 8 bis 12, wobei die Verwendung weiterhin umfasst, dem Individuum BNP oder ein Analogon davon zu verabreichen.

14. Die Verwendung gemäß einem der Ansprüche 8 bis 13, wobei die Verwendung weiterhin umfasst, dem Individuum einen Inhibitor der neutralen Endopeptidase zu verabreichen, oder wobei die Verwendung weiterhin umfasst, dem Individuum BNP oder ein Analogon davon und einen Inhibitor der neutralen Endopeptidase zu verabreichen.

15. Eine pharmazeutische Zusammensetzung, die einen oder mehrere Inhibitoren einer Prolyl-spezifischen Dipeptidylpeptidase und ein natriuretisches Peptid umfasst.

## Revendications

1. Procédé *in vitro* d'inhibition de la dégradation d'un ou plusieurs peptides natriurétiques comprenant un résidu proline pénultième amino-terminal dans un milieu aqueux, comprenant :
la mise en contact dudit milieu aqueux avec un ou plusieurs inhibiteurs d'une dipeptidyl peptidase prolyl-spécifique dans des conditions choisies pour inhiber la perte dudit résidu proline d'un ou plusieurs desdits peptides natriurétiques présents dans ledit échantillon.

2. Procédé selon la revendication 1 où ladite étape de mise en contact inhibe la perte d'un résidu proline pénultième amino-terminal de BNP.

3. Procédé selon la revendication 1 ou 2 où un ou plusieurs desdits inhibiteurs de dipeptidyl peptidase prolyl-spécifique sont des inhibiteurs à petite molécule, ou bien où un ou plusieurs desdits inhibiteurs de dipeptidyl peptidase prolyl-spécifique comprennent des anticorps ou des fragments d'anticorps.

4. Procédé selon la revendication 3 où les un ou plusieurs inhibiteurs de dipeptidyl peptidase prolyl-spécifique comprennent un analogue dipeptidique comprenant Xaa-boroPro, où Xaa peut être tout aminoacide et boroPro est un ester proline boronate.

5. Procédé selon la revendication 3 où les un ou plusieurs inhibiteurs de dipeptidyl peptidase prolyl-spécifique comprennent Diprotin A.

6. Procédé selon l'une quelconque des revendications 1 à 5 où ledit milieu aqueux est le sang, le sérum ou le plasma.

7. Procédé selon l'une quelconque des revendications 1 à 6 comprenant en outre la mise en oeuvre d'un test pour un ou plusieurs peptides natriurétiques présents sur tout ou partie dudit milieu aqueux.

8. Utilisation d'un ou plusieurs inhibiteurs de dipeptidyl peptidase prolyl-spécifique pour la fabrication d'un médicament pour traiter un sujet nécessitant une fonction de peptide natriurétique accrue, où lesdits un ou plusieurs desdits inhibiteurs de dipeptidyl peptidase prolyl-spécifique sont (I) un analogue dipeptidique comprenant Xaa-boroPro, où Xaa peut être tout aminoacide et boroPro est un ester proline boronate, (2) Diprotin A ou (3) des anticorps ou fragments d'anticorps.

9. Utilisation d'un ou plusieurs inhibiteurs de dipeptidyl peptidase prolyl-spécifique pour la fabrication d'un médicament pour inhiber la dégradation d'un peptide natriurétique présent chez un sujet, comprenant l'administration desdits un ou plusieurs inhibiteurs de dipeptidyl peptidase prolyl-spécifique en une quantité suffisante pour inhiber la dégradation du peptide natriurétique par une dipeptidyl peptidase prolyl-spécifique, où lesdits un ou plusieurs desdits inhibiteurs de dipeptidyl peptidase prolyl-spécifique sont (I) un analogue dipeptidique comprenant Xaa-boroPro, où Xaa peut être tout aminoacide et boroPro est un ester proline boronate, (2) Diprotin A ou (3) des anticorps ou fragments d'anticorps.

10. Utilisation selon la revendication 9 comprenant en outre :
la détermination de la présence ou de la quantité d'un ou plusieurs peptides natriurétiques dans un échantillon obtenu auprès dudit sujet ; et
la détermination d'un schéma de traitement sur la base de la présence ou de la quantité desdits un ou plusieurs peptides natriurétiques.

11. Utilisation selon l'une quelconque des revendications 8 à 10 où ledit sujet est choisi pour recevoir lesdits un ou plusieurs inhibiteurs de dipeptidyl peptidase prolyl-spécifique sur la base d'un diagnostic choisi dans le groupe consistant en l'attaque, l'insuffisance cardiaque congestive, l'ischémie cardiaque, l'hypertension systémique et l'infarctus du myocarde aigu.

12. Utilisation selon l'une quelconque des revendications 8 à 11 où ledit sujet est choisi pour recevoir lesdits un ou plusieurs inhibiteurs de dipeptidyl peptidase prolyl-spécifique sur la base de ce qu'il a un niveau plasmatique de BNP d'au moins 80 pg/ml avant l'administration du médicament.

13. Utilisation selon l'une quelconque des revendications 8 à 12 où ladite utilisation comprend en outre l'administration de BNP ou d'un analogue de celui-ci audit sujet.

14. Utilisation selon l'une quelconque des revendications 8 à 13 où ladite utilisation comprend en outre l'administration d'un inhibiteur d'endopeptidase neutre audit sujet ou bien où ladite utilisation comprend en outre l'administration de BNP ou d'un analogue de celui-ci et d'un inhibiteur d'endopeptidase neutre audit sujet.

15. Composition pharmaceutique comprenant un ou plusieurs inhibiteurs de dipeptidyl peptidase prolyl-spécifique et un peptide natriurétique.
